# FASCICULE DE BREVET EUROPEEN

(11) **EP 3 634 551 B1**
(45) Date de publication et mention de la délivrance du brevet: **24.02.2021**
(21) Numéro de dépôt: 18737657.9
(22) Date de dépôt: 04.06.2018
(51) Int. Cl.: A61M 11/02, A61M 15/08, A61M 11/00, A61M 15/00, B05B 11/06

(54) **DISPOSITIF DE DISTRIBUTION DE PRODUIT FLUIDE OU PULVÉRULENT**
SPENDERVORRICHTUNG FÜR FLÜSSIGE ODER PULVERFÖRMIGE PRODUKTE
FLUID OR POWDERY PRODUCT DISPENSING DEVICE

(30) Priorité: 06.06.2017 FR 1754977
(43) Date de publication de la demande: 15.04.2020
(73) Titulaire: APTAR France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2018/051281
(87) Numéro de publication internationale: WO 2018/224762

(56) Documents cités:
- WO-A1-99/34853
- WO-A1-2015/001269
- WO-A1-2015/001281

## Description

La présente invention concerne un dispositif de distribution de produit fluide ou pulvérulent, et plus particulièrement un dispositif pour distribuer unitairement une dose de produit, notamment de poudre, contenue dans un réservoir à l'aide d'un écoulement d'air sous pression.

Le document WO9946055 divulgue un tel dispositif dans lequel un élément de fermeture sphérique, qui obture la sortie du réservoir, est expulsé par l'écoulement d'air créé par une chasse d'air. Pour l'utilisation d'un dispositif de distribution de poudre plus particulièrement, la pression d'air nécessaire pour actionner le dispositif devra être suffisamment élevée pour garantir la distribution complète de la dose, ainsi que son fractionnement si cela est nécessaire. Dans le dispositif susmentionné, la pression d'air nécessaire pour actionner le dispositif est déterminée par la résistance donnée par la bille pour être expulsée. Cette résistance est relativement difficile à contrôler et à prédéterminer puisqu'elle est dépendante du frottement entre la bille et son siège cylindrique dans lequel elle est emmanchée pour obturer de manière étanche ledit réservoir. Il peut par conséquent être nécessaire de minimiser l'interférence entre la sphère et son siège cylindrique, ce qui peut évidemment altérer l'efficacité de l'obturation. De plus, il peut être nécessaire de minimiser la profondeur et le positionnement de la sphère dans son siège afin de faciliter son expulsion. Il peut également être nécessaire de fournir une pression d'air relativement élevée qui n'est pas toujours facile à réaliser à l'aide d'un système de pompe ou d'un système de soufflet, notamment lorsque ces chasses d'air sont actionnées manuellement par le patient. De plus, la distribution, c'est à dire l'expulsion de la bille de son siège, peut se produire à des longueurs différentes de la course de la pompe ou du soufflet de la chasse d'air, de sorte que l'instant précis de la distribution du produit ne peut pas être toujours prédéterminé de manière exacte. Enfin, il y a une limitation dans le choix des matériaux pour la sphère et pour son siège.

Le document WO0245866 décrit un dispositif dans lequel une bille de fermeture est expulsée mécaniquement par une tige solidaire d'une chasse d'air. Ce dispositif n'est pas réutilisable, et l'ensemble du dispositif doit être jeté après son utilisation. Notamment pour des raisons écologiques et économiques, il peut être souhaitable d'avoir un dispositif réutilisable dans lequel le réservoir serait changé après chaque actionnement mais pas la chasse d'air.

Les documents WO2015001269 et WO2015001281 décrivent des dispositifs similaires à celui du document WO0245866, dans lesquels la chasse d'air du dispositif peut être réutilisée avec plusieurs réservoirs de poudre. Ces dispositifs présentent également des inconvénients. Ainsi, ils permettent de réutiliser la chasse d'air, mais pas la tête de distribution qui est jetable avec le réservoir après chaque utilisation. De plus, ces dispositifs requièrent une pièce mobile reliée au réservoir par des ponts sécables, ce qui rend complexe la fabrication du dispositif. Ceci génère de plus un son lors de l'actionnement, lorsque les ponts sécables se brisent, qui peut induire l'utilisateur en erreur. Par ailleurs, les dispositifs des documents WO2015001269, WO2015001281 et WO0245866 comportent une tige qui traverse le réservoir, ce qui limite le volume utile de celui-ci et rend le remplissage du réservoir plus complexe.

La présente invention a pour but de fournir un dispositif de distribution de produit fluide ou pulvérulent qui ne reproduit pas les inconvénients susmentionnés.

La présente invention a notamment pour but de fournir un tel dispositif de distribution de produit fluide ou pulvérulent qui puisse être réutilisé plusieurs fois avec plusieurs réservoirs différents.

La présente invention a aussi pour but de fournir un dispositif de distribution de produit fluide ou pulvérulent, qui soit simple et peu coûteux à fabriquer, à assembler, à remplir et à utiliser.

La présente a donc pour objet un dispositif de distribution de produit fluide ou pulvérulent comportant une tête de distribution pourvue d'une ouverture de distribution, une chasse d'air pour générer un écoulement d'air lors de l'actionnement du dispositif, et au moins un réservoir contenant une dose unique de produit, ledit réservoir comportant une entrée d'air reliée à ladite chasse d'air et une sortie de produit reliée à ladite ouverture de distribution, ledit réservoir étant monté de manière amovible sur ladite chasse d'air, de sorte qu'après l'actionnement du dispositif, le réservoir vide peut être retiré de ladite chasse d'air et remplacé par un nouveau réservoir plein, ladite chasse d'air étant adaptée à revenir en position de repos pour permettre un nouvel actionnement avec ledit nouveau réservoir plein, ladite entrée d'air étant obturée par un premier élément de fermeture et ladite sortie de produit étant obturée par un second élément de fermeture, ledit dispositif comportant un système d'ouverture mécanique coopérant avec lesdits premier et second éléments de fermeture pour les expulser mécaniquement chacun de leur position d'obturation respective lors de l'actionnement du dispositif, ledit système d'ouverture mécanique comportant une première tige solidaire de ladite chasse d'air et une seconde tige solidaire de ladite tête de distribution, lesdites première et seconde tiges coopérant chacune lors de l'actionnement avec un élément de fermeture respectif, pour l'expulser mécaniquement de sa position d'obturation.

Avantageusement, ledit réservoir est symétrique et les deux éléments de fermeture sont identiques.

Avantageusement, ledit réservoir est réalisé, notamment par moulage, en une seule pièce monobloc.

En variante, ledit réservoir est réalisé, notamment par moulage, en deux parties, avantageusement identiques, qui sont fixées l'une à l'autre, notamment soudées ou collées.

Avantageusement, ledit réservoir comporte un corps principal cylindrique se terminant à ses extrémités axiales par ladite entré d'air et ladite sortie de produit.

Avantageusement, ladite entrée d'air et ladite sortie de produit sont formées par des parties cylindriques de diamètre réduit par rapport audit corps principal, permettant une coopération étanche avec lesdits deux éléments de fermeture, qui y sont insérés à force.

Avantageusement, après l'actionnement, lesdits deux éléments de fermeture sont disposés à l'intérieur dudit réservoir.

Avantageusement, ledit réservoir comporte à l'extérieur une bride radiale s'étendant radialement vers l'extérieur, de préférence environ au milieu dudit réservoir, pour fixer ledit réservoir sur ladite chasse d'air.

Avantageusement, ladite bride radiale n'est pas circulaire, mais de forme environ rectangulaire, avec une extension radiale supérieure dans une première direction par rapport à une seconde direction perpendiculaire à ladite première direction.

Avantageusement, ledit premier élément de fermeture et/ou ledit second élément de fermeture est/sont réalisé(s) sous la forme d'une bille.

Avantageusement, ladite chasse d'air comporte un piston coulissant dans une chambre d'air entre une position de repos et une position de distribution, ledit piston, en position de repos, coopérant de manière non étanche avec ladite chambre d'air, de sorte que ladite chambre d'air est reliée à l'atmosphère en position de repos.

Avantageusement, ladite chambre d'air comporte un corps cylindrique comportant à son bord axial supérieur une bride radiale.

Avantageusement, ladite bride radiale comporte sur sa surface supérieure des moyens de fixation dudit réservoir.

Avantageusement, lesdits moyens de fixation comportent des projections formant des épaulements diamétralement opposés adaptés à recevoir et bloquer axialement ladite bride radiale dudit réservoir.

Avantageusement, ladite chasse d'air est ramenée manuellement en position de repos lors du remplacement d'un réservoir vide par un réservoir plein.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section transversale d'un dispositif de distribution de produit fluide ou pulvérulent selon un mode de réalisation avantageux, en position de repos,
- les figures 2 à 5 sont des vues similaires à celle de la figure 1, montrant différentes phases de l'actionnement,
- les figures 6 et 7 sont des vues de détail du réservoir respectivement avant et après actionnement du dispositif,
- les figures 8 et 9 sont des vues de détail du réservoir, montrant deux variantes de fabrication dudit réservoir,
- la figure 10 est une vue de détail en section transversale d'une première variante d'assemblage d'un nouveau réservoir dans le dispositif,
- la figure 11 est une vue en perspective de la première variante d'assemblage de la figure 10,
- la figure 12 est une vue similaire à celle de la figure 10, montrant une seconde variante d'assemblage, et
- la figure 13 est une vue en perspective de la seconde variante d'assemblage de la figure 12.

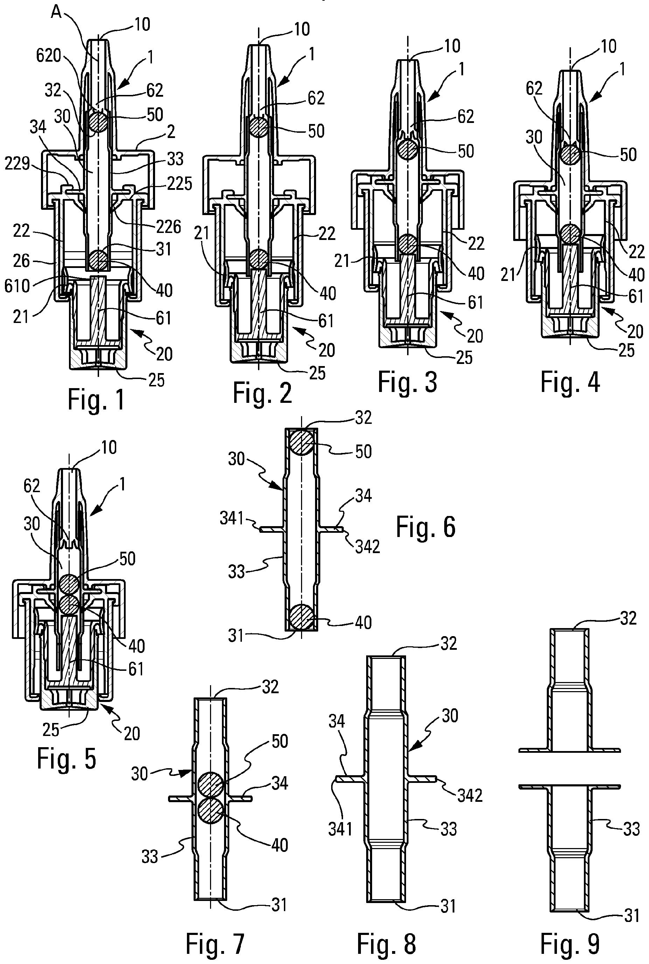

Il est entendu que dans toute la description, les termes "supérieur", "inférieur", "haut" et "bas" se réfèrent à la position droite du dispositif représenté notamment sur la figure 1. Les termes "axial" et "radial" se réfèrent à l'axe central longitudinal A du dispositif, représenté notamment sur la figure 1.

Le dispositif comporte un réservoir 30 contenant une dose de produit à distribuer, ledit réservoir 30 comportant une entrée d'air 31 et une sortie de produit 32. L'entrée d'air 31 du réservoir est reliée à une chasse d'air 20 et la sortie de produit 32 du réservoir est reliée à une ouverture de distribution 10 du dispositif. L'entrée d'air 31 est obturée par un premier élément de fermeture 40, notamment une bille, qui est emmanché à force dans ladite entrée d'air 31. La sortie de produit 32 est obturée par un second élément de fermeture 50, notamment une bille, qui est emmanché à force dans ladite sortie de produit 32. Avant l'actionnement du dispositif, la dose de produit à distribuée est donc maintenue dans ledit réservoir 30, entre lesdits deux éléments de fermeture 40, 50. Ces deux éléments de fermeture 40, 50 assurent de préférence l'étanchéité du produit contenu dans le réservoir.

Avantageusement, comme visible sur les figures 6 à 9, le réservoir 30 est symétrique et les deux éléments de fermeture 40, 50 sont identiques, de sorte que le réservoir peut être utilisé dans ses deux orientations axiales.

Le réservoir 30 peut être réalisé, notamment par moulage, en une seule pièce monobloc, comme représenté sur la figure 8. En variante, il peut être réalisé en deux parties, avantageusement identiques, qui sont ensuite fixées l'une à l'autre, par exemple soudées, notamment par ultrason ou par laser, ou collées.

Le réservoir 30 comporte avantageusement un corps principal cylindrique 33 se terminant à ses extrémités axiales par ladite entré d'air 31 et ladite sortie de produit 32. De préférence, l'entrée d'air 31 et la sortie de produit 32 sont formées par des parties cylindriques de diamètre réduit par rapport audit corps principal 33, permettant une coopération étanche avec les deux éléments de fermeture 40, 50, qui y sont insérés à force, comme illustré sur la figure 6. Lors de l'actionnement, les deux éléments de fermeture 40, 50 sont déplacés vers l'intérieur du réservoir 30, comme illustré sur la figure 7. Le diamètre supérieur dudit corps principal 33 permet alors l'entrée d'un écoulement d'air et l'expulsion du produit au moyen dudit écoulement d'air, malgré la présence des deux éléments de fermeture 40, 50. Réaliser ces deux éléments de fermeture 40, 50 sous forme de billes est ici particulièrement avantageux, pour empêcher toute retenue involontaire de produit dans le réservoir lors de l'expulsion.

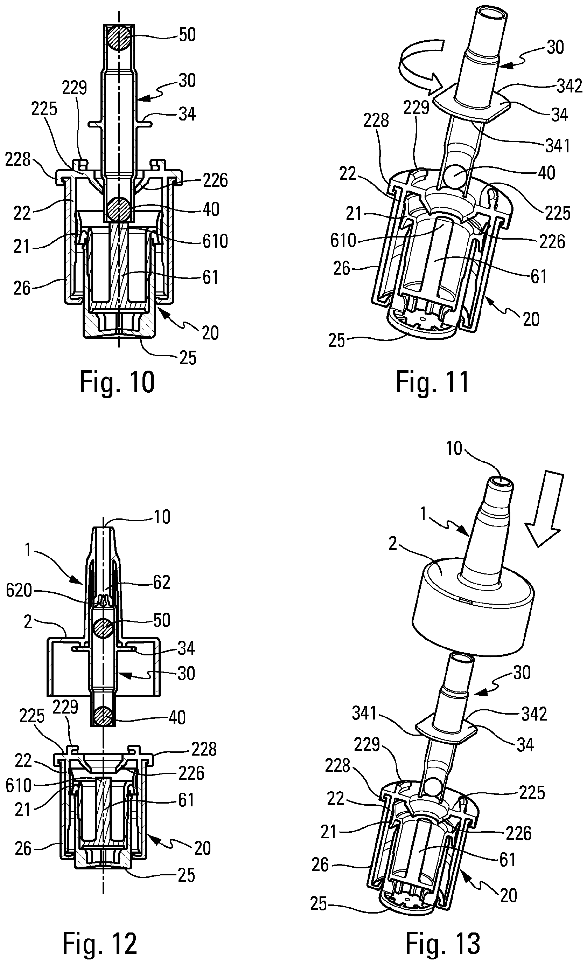

Le réservoir 30 comporte à l'extérieur une bride radiale 34 s'étendant radialement vers l'extérieur, de préférence environ au milieu dudit réservoir 30, pour fixer ledit réservoir 30 sur une chasse d'air 20 comme décrit ci-après. Comme visible notamment sur les figures 11 et 13, cette bride radiale 34 n'est pas circulaire, mais de forme environ rectangulaire, avec une extension radiale supérieure dans une première direction par rapport à une seconde direction perpendiculaire à ladite première direction. Avantageusement, les bords d'extrémité radiale 341, 342 dans ladite première direction sont arrondis. Cette bride radiale 34 sert à fixer de manière amovible le réservoir 30 sur une chasse d'air, comme cela sera plus amplement décrit ci-après.

Le dispositif comporte une chasse d'air 20 qui est actionnée manuellement par l'utilisateur et qui est adaptée à créer un écoulement d'air qui va traverser le réservoir 30 pour emmener le produit qu'il contient en direction de la sortie de distribution 10.

Le réservoir 30 est solidaire, notamment emmanché à force, dans une tête de distribution 1 qui comporte l'ouverture de distribution 10.

Le dispositif comporte un système d'ouverture mécanique 61, 62, qui est adapté à coopérer avec lesdits premier et second éléments de fermeture 40 et 50 pour les expulser mécaniquement de leurs positions d'obturation lors de l'actionnement du dispositif. Selon l'invention, le système d'ouverture mécanique comporte une première tige 61 solidaire de la chasse d'air 20, et une seconde tige 62 solidaire de la tête de distribution 1. Les tiges 61, 62 coopèrent chacune lors de l'actionnement avec un élément de fermeture 40, 50 respectif, pour l'expulser mécaniquement de sa position d'obturation.

Les premier et second éléments de fermeture 40, 50 sont de préférence sphériques, par exemple des billes comme décrit ci-dessus, mais ils pourraient être non sphériques, par exemple de forme ovoïde.

La chasse d'air représentée sur la figure 1 comporte un piston 21 coulissant dans une chambre d'air 22, le piston 21 étant actionné manuellement par l'utilisateur. Avantageusement, cet actionnement est réalisé au moyen d'un élément poussoir 25 assemblé sur ledit piston 21.

Le piston 21 est solidaire de la première tige 61, avantageusement en étant formé sur une même pièce monobloc.

La chambre d'air 22 comporte avantageusement un corps cylindrique comportant à son bord axial supérieur une bride radiale 225. Cette bride radiale 225 s'étend d'une part radialement vers l'intérieur dudit corps cylindrique, où elle comporte une extension axiale 226 s'étendant axialement vers le bas. Cette extension axiale 226 a pour but de coopérer, avantageusement de manière sensiblement étanche, avec la surface externe du réservoir 30, lorsque le dispositif est assemblé. La bride radiale 225 s'étend d'autre part radialement vers l'extérieur dudit corps cylindrique, où elle comporte des moyens de fixation 228 à un corps externe 26 assemblé autour de ladite chambre d'air 22.

Sur sa surface supérieure, ladite bride radiale 225 comporte des moyens de fixation 229 du réservoir 30. Ces moyens de fixation 229 comportent avantageusement des projections formant des épaulements diamétralement opposés adaptés à recevoir et bloquer axialement la bride radiale 34 du réservoir 30. La fixation du réservoir 30 sur la chasse d'air 20 est donc du type baïonnette, avec la bride radiale 34 du réservoir 30 qui vient se placer sur la bride radiale 225 de la chambre d'air 22, puis le réservoir est tourné, typiquement d'environ 90°, pour venir coincer ladite bride radiale 34 sous les projections 229.

En position de repos, visible sur la figure 1, la chasse d'air 20 est avantageusement ouverte sur l'atmosphère.

L'actionnement du dispositif est illustré à titre d'exemple sur les figures 2 à 5. Ainsi, lorsque l'utilisateur souhaite actionner le dispositif, il place d'une part ses doigts sur un repose-doigt 2 de la tête de distribution 1 et d'autre part son pouce sur l'élément poussoir 25, et il exerce une force d'actionnement, qui va déplacer la première tige 61 et le piston 21 vers la position de distribution. Dès le début de l'actionnement, visible sur la figure 2, le piston 21 de la chasse d'air va coopérer de manière étanche avec la chambre d'air 22, de sorte que l'air contenu dans ladite chambre d'air 22 va être progressivement comprimé au cours de l'actionnement.

Dans la position de la figure 2, l'extrémité axiale supérieure 610 de la première tige 61 vient en contact du premier élément de fermeture 40 et l'extrémité axiale inférieure 620 de la seconde tige 62 vient en contact du second élément de fermeture 50.

Une poursuite de l'actionnement déplace d'abord ledit second élément de fermeture 50 vers le bas, comme visible sur les figures 2 et 3. En effet, la première tige 61 étant solidaire du piston 21 de la chasse d'air, elle rencontre une plus forte résistance que la seconde tige 62.

L'actionnement provoque donc la compression de l'air contenu dans la chambre d'air 22, l'ouverture de la sortie de produit 32 par expulsion du second élément de fermeture 50 de sa position d'obturation, et l'ouverture de l'entrée d'air 31 par expulsion du premier élément de fermeture 40 de sa position d'obturation.

Comme visible sur les figures 3 et 4, ledit premier élément de fermeture 40 est poussé axialement vers le haut dans le réservoir 30, donc hors de sa position d'obturation ou de fermeture étanche de l'entrée d'air. A ce moment-là, l'air comprimé dans la chambre d'air 22 peut donc pénétrer dans le réservoir 30.

Une course d'actionnement complète va donc expulser la totalité de la dose de produit contenue dans le réservoir 30 au moyen du flux d'air comprimé crée par la chasse d'air 20, comme visible sur la figure 5.

La présente invention concerne un dispositif rechargeable.

Ainsi, après actionnement, l'utilisateur peut retirer la tête de distribution 1 du réservoir 30 vide et séparer ledit réservoir 30 de la chasse d'air 20 par rotation d'un quart de tour. Après retrait du réservoir vide, illustré sur la figure 10, un nouveau réservoir 30 plein peut être assemblé sur la chasse d'air 20.

Les figures 10 et 11 montrent une première variante dans laquelle le réservoir plein 30 est d'abord assemblé sur la chasse d'air, par translation axiale vers le bas puis rotation d'un quart de tour comme décrit précédemment, puis la tête de distribution 1 est assemblée autour dudit ensemble formé par la chasse d'air 20 et le réservoir 30. Lorsque l'utilisateur insère le réservoir 30 dans la chasse d'air 20, il pousse la première tige 61 vers le bas, ce qui provoque donc le coulissement du piston 21 en retour vers sa position de repos. Pour ce faire, la résistance au retour du piston 21 est inférieure à celle nécessaire pour déplacer ledit premier élément de fermeture 40 hors de sa position d'obturation. En fin d'insertion axiale, le réservoir 30 est tourné d'un quart de tour, de sorte que la bride radiale 34 du réservoir 30 vient coopérer avec les moyens de fixation. Le dispositif est alors prêt pour un nouveau cycle d'actionnement.

Les figures 12 et 13 illustrent une seconde variante, dans laquelle la tête de distribution 1 est d'abord assemblée autour du réservoir 30 plein, puis l'ensemble formé par ladite tête de distribution 1 et ledit réservoir 30 plein est assemblé sur la chasse d'air 20, par translation axiale vers le bas puis rotation d'un quart de tour comme décrit précédemment.

Bien que destiné principalement à la distribution d'un produit pulvérulent, la présente invention s'applique également à la distribution de produits liquides.

La présente invention a été décrite en référence à plusieurs modes de réalisation, mais il est entendu qu'un homme du métier peut y apporter toutes modifications, sans sortir du cadre de la présente invention tel que défini par les revendications annexées.

## Revendications

1. Dispositif de distribution de produit fluide ou pulvérulent comportant une tête de distribution (1) pourvue d'une ouverture de distribution (10), une chasse d'air (20) pour générer un écoulement d'air lors de l'actionnement du dispositif, et au moins un réservoir (30) contenant une dose unique de produit, ledit réservoir (30) comportant une entrée d'air (31) reliée à ladite chasse d'air (20) et une sortie de produit (32) reliée à ladite ouverture de distribution (10), ledit réservoir (30) étant monté de manière amovible sur ladite chasse d'air (20), de sorte qu'après l'actionnement du dispositif, le réservoir vide peut être retiré de ladite chasse d'air et remplacé par un nouveau réservoir plein, ladite chasse d'air (20) étant adaptée à revenir en position de repos pour permettre un nouvel actionnement avec ledit nouveau réservoir plein, ladite entrée d'air (31) étant obturée par un premier élément de fermeture (40) et ladite sortie de produit (32) étant obturée par un second élément de fermeture (50), ledit dispositif comportant un système d'ouverture mécanique (61, 62) coopérant avec lesdits premier et second éléments de fermeture (40, 50) pour les expulser mécaniquement chacun de leur position d'obturation respective lors de l'actionnement du dispositif, **caractérisé en ce que** ledit système d'ouverture mécanique comporte une première tige (61) solidaire de ladite chasse d'air (20) et une seconde tige (62) solidaire de ladite tête de distribution (1), lesdites première et seconde tiges (61, 62) coopérant chacune lors de l'actionnement avec un élément de fermeture (40, 50) respectif, pour l'expulser mécaniquement de sa position d'obturation.

2. Dispositif selon la revendication 1, dans lequel ledit réservoir (30) est symétrique et les deux éléments de fermeture (40, 50) sont identiques.

3. Dispositif selon la revendication 1 ou 2, dans lequel ledit réservoir (30) est réalisé, notamment par moulage, en une seule pièce monobloc.

4. Dispositif selon la revendication 1 ou 2, dans lequel ledit réservoir (30) est réalisé, notamment par moulage, en deux parties, avantageusement identiques, qui sont fixées l'une à l'autre, notamment soudées ou collées.

5. Dispositif selon l'une quelconque des revendications précédentes, dans lequel ledit réservoir (30) comporte un corps principal cylindrique (33) se terminant à ses extrémités axiales par ladite entré d'air (31) et ladite sortie de produit (32).

6. Dispositif selon la revendication 5, dans lequel ladite entrée d'air (31) et ladite sortie de produit (32) sont formées par des parties cylindriques de diamètre réduit par rapport audit corps principal (33), permettant une coopération étanche avec lesdits deux éléments de fermeture (40, 50), qui y sont insérés à force.

7. Dispositif selon l'une quelconque des revendications précédentes, dans lequel, après l'actionnement, lesdits deux éléments de fermeture (40, 50) sont disposés à l'intérieur dudit réservoir (30).

8. Dispositif selon l'une quelconque des revendications précédentes, dans lequel ledit réservoir (30) comporte à l'extérieur une bride radiale (34) s'étendant radialement vers l'extérieur, de préférence environ au milieu dudit réservoir (30), pour fixer ledit réservoir (30) sur ladite chasse d'air (20).

9. Dispositif selon la revendication 8, dans lequel ladite bride radiale (34) n'est pas circulaire, mais de forme environ rectangulaire, avec une extension radiale supérieure dans une première direction par rapport à une seconde direction perpendiculaire à ladite première direction.

10. Dispositif selon l'une quelconque des revendications précédentes, dans lequel ledit premier élément de fermeture (40) et/ou ledit second élément de fermeture (50) est/sont réalisé(s) sous la forme d'une bille.

11. Dispositif selon l'une quelconque des revendications précédentes, dans lequel ladite chasse d'air comporte un piston (21) coulissant dans une chambre d'air (22) entre une position de repos et une position de distribution, ledit piston (21), en position de repos, coopérant de manière non étanche avec ladite chambre d'air (22), de sorte que ladite chambre d'air (22) est reliée à l'atmosphère en position de repos.

12. Dispositif selon la revendication 11, dans lequel ladite chambre d'air (22) comporte un corps cylindrique comportant à son bord axial supérieur une bride radiale (225).

13. Dispositif selon la revendication 12, dans lequel ladite bride radiale (225) comporte sur sa surface supérieure des moyens de fixation (229) dudit réservoir (30).

14. Dispositif selon l'une des revendications 8 et 9 et selon la revendication 13, dans lequel lesdits moyens de fixation (229) comportent des projections formant des épaulements diamétralement opposés adaptés à recevoir et bloquer axialement ladite bride radiale (34) dudit réservoir (30).

15. Dispositif selon l'une quelconque des revendications précédentes, dans lequel ladite chasse d'air est ramenée manuellement en position de repos lors du remplacement d'un réservoir vide par un réservoir plein.

## Patentansprüche

1. Spendervorrichtung für ein flüssiges oder pulverförmiges Produkt, umfassend einen Spenderkopf (1), der mit einer Abgabeöffnung (10) versehen ist, eine Luftstoßeinrichtung (20) zur Erzeugung eines Luftstroms bei Betätigung der Vorrichtung, und mindestens einen Behälter (30), der eine Einzeldosis des Produkts enthält, wobei der Behälter (30) einen Lufteinlass (31), der mit der Luftstoßeinrichtung (20) verbunden ist, und einen Produktauslass (32), der mit der Abgabeöffnung (10) verbunden ist, umfasst, wobei der Behälter (30) abnehmbar an der Luftstoßeinrichtung (20) angebracht ist, sodass nach Betätigung der Vorrichtung der leere Behälter aus der Luftstoßeinrichtung entfernt und durch einen neuen vollen Behälter ersetzt werden kann, wobei die Luftstoßeinrichtung (20) so ausgelegt ist, dass sie in eine Ruheposition zurückkehrt, um eine neue Betätigung mit dem neuen vollen Behälter zu ermöglichen, wobei der Lufteinlass (31) durch ein erstes Verschlusselement (40) verschlossen ist und der Produktauslass (32) durch ein zweites Verschlusselement (50) verschlossen ist, wobei die Vorrichtung ein mechanisches Öffnungssystem (61, 62) umfasst, das mit dem ersten und dem zweiten Verschlusselement (40, 50) zusammenwirkt, um sie bei Betätigung der Vorrichtung jeweils mechanisch aus ihrer Verschlussstellung auszustoßen, **dadurch gekennzeichnet, dass** das mechanische Öffnungssystem eine erste Stange (61), die einstückig mit der Luftstoßeinrichtung (20) ausgebildet ist, und eine zweite Stange (62), die einstückig mit dem Spenderkopf (1) ausgebildet ist, umfasst, wobei die erste und die zweite Stange (61, 62) jeweils bei Betätigung mit einem entsprechenden Verschlusselement (40, 50) zusammenwirken, um dieses mechanisch aus seiner Verschlussstellung auszustoßen.

2. Vorrichtung nach Anspruch 1, bei der der Behälter (30) symmetrisch ist und die beiden Verschlusselemente (40, 50) identisch sind.

3. Vorrichtung nach Anspruch 1 oder 2, bei der der Behälter (30) insbesondere durch einen integralen Formvorgang einstückig hergestellt ist.

4. Vorrichtung nach Anspruch 1 oder 2, bei der der Behälter (30) insbesondere durch eine Formvorgang aus zwei vorzugsweise identischen Teilen hergestellt ist, die aneinander befestigt, insbesondere miteinander verschweißt oder verklebt, sind.

5. Vorrichtung nach einem der vorstehenden Ansprüche, bei der der Behälter (30) einen zylindrischen Hauptkörper (33) aufweist, der an seinen axialen Enden jeweils mit dem Lufteinlass (31) bzw. mit dem Produktauslass (32) endet.

6. Vorrichtung nach Anspruch 5, bei der der Lufteinlass (31) und der Produktauslass (32) aus zylindrischen Abschnitten mit in Bezug auf den Hauptkörper (33) verringertem Durchmesser gebildet sind, um eine abgedichtete Verbindung mit den beiden Verschlusselementen (40, 50) zu ermöglichen, die darin eingepresst sind.

7. Vorrichtung nach einem der vorstehenden Ansprüche, bei der nach ihrer Betätigung die beiden Verschlusselemente (40, 50) im Inneren des Behälters (30) angeordnet sind.

8. Vorrichtung nach einem der vorstehenden Ansprüche, bei der der Behälter (30) an der Außenseite einen radialen Flansch (34) aufweist, der radial nach außen verläuft, vorzugsweise ungefähr in der Mitte des Behälters (30), um den Behälter (30) an der Luftstoßeinrichtung (20) zu befestigen.

9. Vorrichtung nach Anspruch 8, bei der der radiale Flansch (34) nicht kreisförmig ist, sondern eine annähernd rechteckige Form hat, mit einer größeren radialen Erstreckung in einer ersten Richtung relativ zu einer zweiten Richtung, die senkrecht zu der ersten Richtung ist.

10. Vorrichtung nach einem der vorstehenden Ansprüche, bei der das erste Verschlusselement (40) und/oder das zweite Verschlusselement (50) kugelförmig ausgebildet ist/sind.

11. Vorrichtung nach einem der vorstehenden Ansprüche, bei der die Luftstoßeinrichtung einen Kolben (21) aufweist, der in einer Luftkammer (22) zwischen einer Ruhestellung und einer Abgabestellung gleitet, wobei der Kolben (21) in seiner Ruhestellung nicht abdichtend mit der Luftkammer (22) zusammenwirkt, sodass die Luftkammer (22) in der Ruhestellung mit der Atmosphäre verbunden ist.

12. Vorrichtung nach Anspruch 11, bei der die Luftkammer (22) einen zylindrischen Körper umfasst, der an seinem oberen axialen Rand einen radialen Flansch (225) aufweist.

13. Vorrichtung nach Anspruch 12, bei der der radiale Flansch (225) an seiner Oberseite Mittel (229) zur Befestigung des Behälters (30) aufweist.

14. Vorrichtung nach einem der Ansprüche 8 und 9 und nach Anspruch 13, bei der die Befestigungsmittel (229) Vorsprünge aufweisen, die diametral gegenüberliegende Schultern bilden, die geeignet sind, den radialen Flansch (34) des Behälters (30) aufzunehmen und axial zu fixieren.

15. Vorrichtung nach einem der vorstehenden Ansprüche, bei der bei Austausch eines leeren Behälters gegen einen vollen Behälter die Rückstellung der Luftstoßeinrichtung in die Ruhestellung von Hand erfolgt.

## Claims

1. A dispenser device for dispensing a fluid or powder composition, the dispenser device including a dispenser head (1) provided with a dispenser opening (10), an air expeller (20) for generating a flow of air while the device is being actuated, and at least one reservoir (30) that contains a single dose of composition, said reservoir (30) including an air inlet (31) that is connected to said air expeller (20), and a composition outlet (32) that is connected to said dispenser opening (10), said reservoir (30) being mounted in removable manner on said air expeller (20) such that, after the device has been actuated, the empty reservoir can be removed from said air expeller and replaced with a new full reservoir, said air expeller (20) being adapted to return to its rest position so as to make a new actuation possible with said new full reservoir, said air inlet (31) being closed by a first closure element (40) and said composition outlet (32) being closed by a second closure element (50), said device including a mechanical opening system (61, 62) that co-operates with said first and second closure elements (40, 50) so as to expel each closure element mechanically from its respective closed position while the device is being actuated, the device being **characterized in that** said mechanical opening system comprises a first rod (61) that is secured to said air expeller (20) and a second rod (62) that is secured to said dispenser head (1), each of said first and second rods (61, 62) co-operating with a respective closure element (40, 50) during actuation, so as to expel it mechanically from its closed position.

2. A device according to claim 1, wherein said reservoir is symmetrical and the two closure elements (40, 50) are identical.

3. A device according to claim 1 or claim 2, wherein said reservoir (30) is made as a single piece, in particular by molding.

4. A device according to claim 1 or claim 2, wherein said reservoir (30) is made as two advantageous identical portions, in particular by molding, which portions are fastened together, in particular by welding or by adhesive bonding.

5. A device according to any preceding claim, wherein said reservoir (30) comprises a cylindrical main body (33) that is terminated at its axial ends by said air inlet (31) and by said composition outlet (32).

6. A device according to claim 5, wherein said air inlet (31) and said composition outlet (32) are formed by cylindrical portions of diameter that is small relative to said main body (33), thereby enabling them to co-operate in sealed manner with said two closure elements (40, 50) that are forced-fitted therein.

7. A device according to any preceding claim, wherein, after actuation, said two closure elements (40, 50) are arranged inside said reservoir (30).

8. A device according to any preceding claim, wherein the outside of said reservoir (30) includes a radial flange (34) that extends radially outwards, preferably from approximately the middle of said reservoir (30), so as to fasten said reservoir (30) on said air expeller (20).

9. A device according to claim 8, wherein said radial flange (34) is not circular, but of shape that is approximately rectangular, having a top radial extent in a first direction that is greater than its radial extent in a second direction perpendicular to said first direction.

10. A device according to any preceding claim, wherein said first closure element (40) and/or said second closure element (50) is/are made as a ball.

11. A device according to any preceding claim, wherein said air expeller includes a piston (21) that slides in an air chamber (22) between a rest position and a dispensing position, said piston (21), when in its rest position, co-operating in non-airtight manner with said air chamber (22), in such a manner that said air chamber (22) is in communication with the atmosphere in the rest position.

12. A device according to claim 11, wherein said air chamber (22) comprises a cylindrical body that includes a radial flange (225) at its top axial edge.

13. A device according to claim 12, wherein, on its top surface, said radial flange (225) includes fastener means (229) for fastening said reservoir (30).

14. A device according to either one of claims 8 and 9 and according to claim 13, wherein said fastener means (229) comprise projections that form diametrically-opposite shoulders that are adapted to receive said radial flange (34) of said reservoir (30) and to hold it in position axially.

15. A device according to any preceding claim, wherein said air expeller is returned manually into its rest position while replacing an empty reservoir with a full reservoir.
